(19) Europäisches Patentamt
European Patent Office
Office européen des brevets

(11) Veröffentlichungsnummer: **0 069 952**
**A2**

(12) # EUROPÄISCHE PATENTANMELDUNG

(21) Anmeldenummer: 82105963.1

(22) Anmeldetag: 03.07.82

(51) Int. Cl.³: **A 61 K 7/06**

(30) Priorität: 09.07.81 DE 3127128

(43) Veröffentlichungstag der Anmeldung: 19.01.83 Patentblatt 83/3

(84) Benannte Vertragsstaaten: CH DE FR GB IT LI NL SE

(71) Anmelder: HOECHST AKTIENGESELLSCHAFT
Postfach 80 03 20
D-6230 Frankfurt/Main 80(DE)

(72) Erfinder: Hartung, Herbert, Dr.
Hochriesstrasse 1
D-8261 Burgkirchen/Alz(DE)

(72) Erfinder: Reng, Alwin
Im Schulzehnten 22
D-6233 Kelkheim(Taunus)(DE)

(72) Erfinder: Quack, Jochen Meinhard, Dr.
Wilhelm-Reuter-Strasse 16
D-6239 Eppstein/Taunus(DE)

(54) **Haarbehandlungsmittel mit quartären Isostearyl-ammoniumverbindungen.**

(57) Haarbehandlungsmittel mit einem Gehalt einer quartären Isostearyl-ammonium-Verbindung der Formel

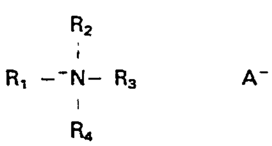

wobei $R_1$ Isostearyl, $R_2$ Isostearyl, $C_1$-$C_4$-Alkyl oder Benzyl, $R_3$ und $R_4$ $C_1$-$C_4$-Alkyl oder Hydroxiethyl und A ein Anion bedeutet.

EP 0 069 952 A2

0069952

HOECHST AKTIENGESELLSCHAFT HOE 81/F 165 Dr.OT/Wa

Haarbehandlungsmittel mit quartären Isostearyl-ammoniumverbindungen

Humanhaare können durch physikalische, chemische und mikrobiologische exogene Einflüsse geschädigt werden; vor allem durch Kämmen und Bürsten wird das Haar irreparabel mechanisch angegriffen. Zur Prophylaxe und zur kosmetischen Nachbehandlung werden deshalb Haarspülmittel, die kationische Tenside (Quats) enthalten, verwendet. Diese Quats werden meist in Kombination mit nichtionischen Emulgatoren bzw. konsistenzgebenden Fettalkoholen in wäßriger Lösung bzw. Suspension angewandt und verbessern nach dem Aufbringen auf die Haare sowohl die Naßkämmerbarkeit durch Herabsetzung der Reibung zwischen Haar/Haar bzw. Haar/Kamm bzw. Bürste und bewirken außerdem eine Reihe von speziellen Eigenschaften wie Glanz, Antistatik und Trockenkämmbarkeit. Häufig verwendete quartäre Ammoniumverbindungen für diesen Zweck sind Cetyltrimethylammoniumchlorid oder -bromid, Stearyldimethylbenzylammoniumchlorid und Distearyldimethylammoniumchlorid .

Es wurde nun gefunden, daß quartäre Ammoniumverbindungen mit einer oder mehreren Isostearyl-Gruppen besondere Vorteile im Hinblick auf Emulgierverhalten, Verteilbarkeit auf den Humanhaaren und auch bezüglich der Haarnaßkämmbarkeit und Schaumarmut bieten.

Gegenstand der Erfindung sind somit Haarbehandlungsmittel, gekennzeichnet durch einen Gehalt einer quartären Isostearyl-ammoniumverbindung der Formel

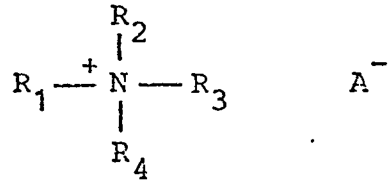

wobei $R_1$ Isostearyl, $R_2$ Isostearyl, $C_1$-$C_4$-Alkyl, vorzugsweise Methyl oder Benzyl, $R_3$ und $R_4$ $C_1$-$C_4$-Alkyl, vorzugsweise Methyl oder Hydroxiethyl und A ein Halogenid-, Methosulfat-,*Methophosphat- oder Ethophosphation bedeutet. *Ethosulfat,

Die quartären Ammoniumverbindungen der obigen Formel werden erhalten nach bekannten Verfahren ausgehend von Isostearinsäure. Aus dieser Säure stellt man mit Ammoniak das Isostearylnitril her, das durch Hydrieren in das Isostearylamin überführt wird. Dieses Isostearylamin wird dann nach bekannten Verfahren alkyliert bzw. quaterniert. Bei der Isostearylgruppe handelt es sich um ein Gemisch von methyl-verzweigten $C_{17}$-Alkylgruppen.

Die quartären Ammoniumverbindungen der obigen Formel weisen technisch interessante oberflächenaktive Eigenschaften auf und eignen sich besonders zur Verwendung in Haarpflegemitteln. So lassen sich durch einfaches Mischen mit Wasser flüssige oder pastöse Haarpflegemittel mit sehr guter Lagerstabilität herstellen. Diese Haarpflegemittel können weiterhin alle solche Verbindungen enthalten, die üblicherweise in diesen Produkten eingesetzt werden, wie etwa Parfümöl, Verdicker, Haarfestiger, Alkohole,antistatisch wirkende Verbindungen und weitere oberflächenaktive Verbindungen. Der Gehalt der quartären Isostearyl-ammonium-Verbindungen in den Haarpflegemitteln ist gleich groß wie bei der Verwendung bisher gebräuchlicher quartärer Ammoniumverbindungen und beträgt ca. 0,1 - 20,vorzugsweise 0,1 - 10 Gew %. Störende Überschüsse der quartären Ammoniumverbindung lassen sich ohne Probleme aus dem Haar auswaschen und durch die gute Verteilung wird auch eine sehr gute Naßkämmbarkeit erreicht. Auch die Trockenkämmbarkeit ist gut.

Die Flüssigeinstellungen der quartären Ätheramine zeigen außerdem nur eine sehr schwache Schaumwirkung. Dies ist von besonderem Vorteil beim Abfüllen der fertigen Haarpflegemittel mit automatischen Dosieranlagen, wo in der gleichen Zeiteinheit eine wesentlich größere Menge an flüssigen Haarpflegemitteln abgefüllt werden kann im Vergleich zu einer schäumenden, wäßrigen Cetyltrimethylammoniumbromid-Lösung. Auch die bei der Anwendung beispielsweise von wäßrigen Cetyltrimethylammoniumchlorid auftretenden Schaumbildungen, die die Frisierbarkeit des Haares beeinträchtigen, sind bei der Verwendung wäßriger quartärer Ätheramin-Lösungen gemäß der vorliegenden Erfindung nicht vorhanden.

Die Herstellung der quartären Ammoniumverbindungen wird in den folgenden Beispielen weiter erläutert. Sofern nicht anders angegeben, beziehen sich alle Mengenangaben auf das Gewicht.

Beispiel 1

a) Herstellung des Isostearylnitrils

In einem Vierhalskolben mit Rührer, Thermometer, Wasserabscheider und Gaseinleitungsrohr werden 2000 g Isostearinsäure und 20 g Zink-dodecylbenzolsulfonat vorgelegt und unter schwachem Stickstoffstrom auf 150°C erhitzt. Nun beginnt man mit dem Einleiten von 40 Liter $NH_3$/Std. und Aufheizen auf 290°C. Nach 12 Stunden Reaktion bei 290°C wird unter Stickstoff abgekühlt und im Vakuum bei 1 - 0,3 mbar destilliert. Man erhält ca. 85 % Isostearylnitril vom Kp 155 - 190°C/ 1 - 0,3 mbar.

b) Herstellung des Isostearylamins

1300 g Isostearylnitril werden zusammen mit 70 g wasserfreiem Raney-Nickel in einen 5 l Schüttelautoklaven gefüllt, 2 x mit Stickstoff gespült und mit 180 bar Wasserstoff auf Dichtigkeit geprüft. Nach Entspannen werden 400 ml flüssiges Ammoniak zugefahren und 80 bar Wasserstoff aufgepreßt. Die Reaktionstemperatur wird auf 80 - 120°C gebracht und der Wasserstoffdruck durch Nachpressen bei 150 - 180 bar gehalten. Nach Erreichen von 180 bar Wasserstoffdruck wird 2 Stunden nachreagiert, auf 50°C abgekühlt und über ein Druckfilter filtriert.

Nach Destillation im Hochvakuum erhält man 1189 g (91,5 %) des bei 142 - 60°C/0,3 mbar siedenden Isostearylamins, das 99,9 % Primäramin-Anteile aufweist.

c) Herstellung des Isostearyltrimethylammoniumchlorids

Eine Mischung von 300 g Isostearylamin, 90 g 50 %iger NaOH und 200 g Isopropanol wird in einem 1 l Glasautoklaven vorgelegt, 2 x mit Stickstoff gespült und auf Dichtigkeit geprüft. Anschließend wird auf ca. 80°C aufgeheizt und mit 170 g flüssigem Methylchlorid umgesetzt. Nach 2 Stunden Reaktion bei 30°C werden weitere 110 g 50 %iger NaOH zugegeben. Nach insgesamt 6,5 Stunden Reaktionszeit werden nochmals 10 g 50 %ige NaOH nachgegeben und weitere 2 Stunden bei 80°C umgesetzt.

Nach Entspannen des überschüssigen Methylchlorids wird der pH-Wert der Reaktionsmischung auf 6 - 7 eingestellt und der Rest Methylchlorid am Rotationsverdampfer entfernt. Nach Absaugen des ausgefällten

NaCl und Zugabe von 8 g Wasser und 15 g Isopropanol werden 505 g Isostearyltrimethylammoniumchlorid 75 %ig in Isopropanol erhalten.

Beispiel 2

a) Herstellung von Diisostearylamin

400 g Isostearylamin werden in einem Rührkolben mit Thermometer und Gaseinleitungsrohr mit 20 g Raney-Nickel versetzt und 100 l Wasserstoff/Stunde eingegast. Die Reaktionstemperatur wird in ca. 30 Minuten auf 160°C gebracht und 1,5 Stunden gehalten. Nach Abkühlen auf ca. 50°C wird der Katalysator abfiltriert und dabei Diisostearylamin in praktisch quantitativer Ausbeute erhalten. Das Reaktionsprodukt enthält neben 92,3 % Sekundär-Anteilen 4,7 % bzw. 4,3 % Primär- und Tertiär-Aminanteile.

b) Herstellung von Diisostearyldimethylammoniumchlorid

126 g Diisostearylamin und 25 g 50 %ige NaOH werden zusammen mit 100 g Isopropanol in einem 1 l-Glasautoklaven mit 28 g flüssigem Methylchlorid analog 1 c umgesetzt. Dabei entsteht ein Methylchlorid-Druck von ca. 5 bar. Nach ca. 2 Stunden Reaktionszeit wird 1 g 50 %ige NaOH nachgegeben und weitere 1,5 Stunden bei 80°C umgesetzt.

Nach Entspannen des überschüssigen Methylchlorids wird der pH-Wert der Reaktionsmischung auf 6 - 7 eingestellt und der Restmethylchlorid am Rotationsverdampfer entfernt. Nach Absaugen des ausgefällten NaCl und Zugabe von 8 g Wasser und 15 g Isopropanol werden ca. 150 g Diisostearyldimethylammoniumchlorid 75 %ig in Isopropanol-Wasser (1:2) erhalten.

Die anwendungstechnischen Eigenschaften der quartären Ammoniumverbindungen mit Isoalkyl-Gruppen im Molekül wurden sowohl in-vivo am Haar von jeweils vier Versuchspersonen als auch in-vitro an Strähnen von mitteleuropäischem Haar beurteilt.

Zunächst wurden jeweils die Haare mit einer 1 %igen Lauryl-diglykolethersulfat-Natriumsalzlösung in Wasser gewaschen und anschließend mit Wasser von 35°C ausgespült. Beim in-vivo-Test wurden 10 ml einer 2 %igen wäßrigen Lösung bzw. Dispersion der quartären Ammoniumverbindung auf das noch feuchte Kopfhaar verteilt und nach 5minütiger Einwirkungszeit wurde mit 2 l Wasser von 35°C die überschüssige Menge der quartären Ammoniumverbindung ausgespült.

Beim in-vitro-Test wurde eine Haarsträhne mit einer Länge von 15 cm und einem Durchmesser von 1,5 cm 3 Minuten in 100 ml einer 2 %igen wäßrigen Lösung bzw. Dispersion der quartären Ammoniumverbindung getaucht. Anschließend wurde die Haarsträhne mit 300 ml Wasser von 35°C ausgespült.

Sowohl am Lebendhaar als auch an den Haarsträhnen wurden eine Reihe von Prüfungen durchgeführt, die nachstehend kurz erläutert sind.

1. <u>Schaumvermögen</u>

Das Schaumvermögen wurde während des Auftragens und Ausspülens subjektiv beurteilt und im allgemeinen wird ein möglichst schaumarmes Verhalten gewünscht.

2. <u>Naßkämmbarkeit</u>

Die Prüfung erfolgt durch Kämmen des nassen Haares,

wobei der Reibungswiderstand zwischen Haar und Kamm möglichst gering sein sollte.

3. Trockenkämmbarkeit

Nach dem Trocknen der behandelten Haare wurden diese bei 25°C und 65 % Luftfeuchte mit einem Haar-Hartgummikamm gekämmt und gleichzeitig auch die antistatische Wirkung beurteilt.

4. Glanz

Der Glanz der Haare wurde bei Verwendung einer konstanten Lichtquelle visuell beurteilt.

5. Spreitung auf den Haaren

Die Verteilbarkeit der Haarbehandlungsmittel auf dem feuchten bzw. trockenen Haar ist von großer Bedeutung für die Effektivität der Produkte. Die Beurteilung erfolgte visuell während der Anwendung und gleichzeitig durch Aufbringen der Haarbehandlungsflüssigkeit auf Filterpapier.

In der Tabelle 1 sind die ermittelten Ergebnisse dargestellt und es zeigt sich, daß die quartären Ammoniumverbindungen mit Isostearylgruppen gegenüber den Vergleichssubstanzen vor allem Vorteile im Hinblick auf die relevanten haarkosmetischen Eigenschaften bieten.

Die folgenden Anwendungsbeispiele veranschaulichen die Einsatzmöglichkeiten der quartären Ammoniumverbindungen mit Isostearylalkylresten zur Herstellung von Haarbehandlungsmitteln.

Die Mengen- und Prozentangaben in den Beispielen beziehen sich auf das Gewicht.

Beispiel 3

Klares, niedrig-viskoses Haarnachspülmittel

2,0 % Isostearyltrimethylammoniumchlorid
ad 100,0 % Wasser.

Beispiel 4

Klares, hoch-viskoses flüssiges Haarnachspülmittel

1,5 % Isostearyltrimethylammoniumchlorid
1,2 % Hydroxyethylcellulose
0,2 % Parfümöl
ad 100,0 % Wasser.

Beispiel 5

Emulsionsförmiges Haarnachspülmittel

2,0 % Isostearyldimethylbenzylammoniumchlorid
3,0 % Stearylalkohol + 3 Mol Ethylenoxid
1,0 % Cetylalkohol
0,2 % Parfümöl
ad 100,0 % Wasser.

Beispiel 6

Cremeförmiges Haarnachspülmittel

2,0 % Diisostearyltrimethylammoniumchlorid
3,0 % Cetylalkohol
2,0 % Triethylenglykoldistearat
1,0 % Stearylalkohol
0,1 % Parfümöl
ad 100,0 % Wasser.

Beispiel 7

Emulsionsförmiges Haarnachspülmittel

1,0 % Cetyltrimethylammoniumchlorid
2,0 % Diisostearyltrimethylammoniumchlorid
1,0 % Diglycerinisostearat
3,0 % Cetylalkohol
ad 100,0 % Wasser.

Beispiel 8

Emulsionsförmiges Haarnachspülmittel

2,0 % Isostearyltrimethylammoniumchlorid
2,0 % Behenyltrimethylammoniumchlorid
3,0 % Cetylalkohol
0,2 % Parfümöl
ad 100,0 % Wasser.

Beispiel 9

Emulsionsförmiges Haarnachspülmittel

3,0 % Isostearyltrimethylammoniumchlorid
1,0 % Lauryl-tetraglykol-orthophosphorsäureester
3,0 % Cetylalkohol
2,0 % Paraffinöl
1,0 % Lanolin
0,2 % Parfümöl
ad 100,0 % Wasser.

Beispiel 10

Frisiercreme

1,0 % Diisostearyltrimethylammoniumchlorid
5,0 % Diglycerinsesquioleat
15,0 % Paraffinwachs

15,0 % Vaseline
0,1 % Parfümöl
ad 100,0 % Wasser.

Beispiel 11

Haarspray

0,2 % Isostearyltrimethylammoniumchlorid
1,5 % Polyvinylpyrrolidon/Vinylacetat Mischpolymerisat, Verhältnis 70:30
0,1 % Parfümöl
28,0 % Ethylalkohol
ad 100,0 % fluorierte Kohlenwasserstoffe als Treibgas

Beispiel 12

Shampoo

2,0 % Isostearyltrimethylammoniumchlorid
15,0 % Lauryldiglykolethersulfat-Natriumsalz
3,0 % Cocosfettsäurediethanolamid
1,3 % Natriumchlorid
0,05 % Formaldehyd
0,2 % Parfümöl
ad 100,0 % Wasser.

Beispiel 13

Flüssiger Haarfestiger

3,0 % Vinylpyrrolidon/Vinylacetat Mischpolymerisat, Verhältnis 60:40
0,15 % Isostearyltrimethylammoniumchlorid
45,0 % Isopropylalkohol
ad 100,0 % Wasser.

TABELLE 1

| PRODUKT | Schaum-armut | Naß-kämm-barkeit | Trocken-kämmbarkeit/ Antistatik | Glanz der Haare | Spreitung auf den Haaren |
|---|---|---|---|---|---|
| Stearyltrimethyl-ammoniumchlorid | 3 | 3 | 3 | 4 | 5 |
| Cetyltrimethyl-ammoniumchlorid | 5 | 3 | 3 | 3 | 4 |
| Isostearyltrimethyl-ammoniumchlorid | 2 | 1 | 1 | 1 | 1 |
| Di-isostearyl-trimethylammoniumchlorid | 1 | 2 | 2 | 2 | 1 |

1 = sehr gut
2 = gut
3 = mäßig
4 = schlecht
5 = sehr schlecht

PATENTANSPRÜCHE:

1. Haarbehandlungsmittel, gekennzeichnet durch einen Gehalt einer quartären Isostearyl-ammonium-Verbindung der Formel

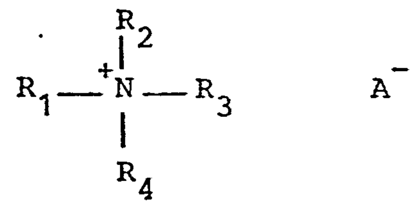

wobei $R_1$ Isostearyl, $R_2$ Isostearyl, $C_1$-$C_4$-Alkyl oder Benzyl, $R_3$ und $R_4$ $C_1$-$C_4$-Alkyl oder Hydroxiethyl und A ein Halogenid-, Methosulfat-, Ethosulfat-, Methophosphat- oder Ethophospation bedeutet.

2. Haarbehandlungsmittel nach Anspruch 1, gekennzeichnet durch einen Gehalt einer quartären Isostearyl-ammonium-Verbindung der Formel

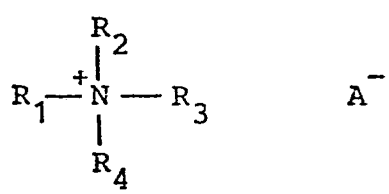

wobei $R_1$ Isostearyl, $R_2$ Isostearyl oder Methyl, $R_3$ und $R_4$ Methyl und A ein Chloridion bedeutet.